# EUROPÄISCHE PATENTSCHRIFT

(11) **EP 1 596 736 B1**
(45) Veröffentlichungstag und Bekanntmachung des Hinweises auf die Patenterteilung: **30.08.2006**
(21) Anmeldenummer: 03789052.2
(22) Anmeldetag: 18.11.2003
(51) Int. Cl.: A61B 17/58

(54) **INJEKTIONSPUMPE**
INJECTION PUMP
POMPE D'INJECTION

(43) Veröffentlichungstag der Anmeldung: 23.11.2005
(73) Patentinhaber: SOMATEX Medical Technologies GmbH, 14513 Teltow (DE)
(72) Erfinder: DUNKER, Thomas, 14513 Teltow (DE); HORNSCHEIDT, Dirk, 10245 Berlin (DE); KNIEP, Frank, 14979 Grossbeeren/ OT Kleinbeeren (DE)
(74) Vertreter: Haschick, Gerald
(86) Internationale Anmeldenummer: PCT/EP2003/012888
(87) Internationale Veröffentlichungsnummer: WO 2005/051212

(56) Entgegenhaltungen:
- DE-A- 10 042 423
- DE-U- 8 908 719
- US-B1- 6 383 190
- US-B1- 6 395 007

## Beschreibung

Die Erfindung betrifft eine Injektionspumpe zum Applizieren von hochviskosen Medien, welche mit hohem Druck appliziert werden müssen, insbesondere bei der perkutanen Vertebroplastie.

Bei der Vertebroplastie handelt es sich um ein Behandlungsverfahren für Knochenschmerzen, welche insbesondere bei Erkrankungen der Wirbelsäule oder anderer Knochen entstehen können. Zu diesen Krankheiten gehören in erster Linie die Osteoporose sowie der Befall durch Tumorerkrankungen. Bei diesen Erkrankungsgruppen wird der Schmerz durch einen zunehmenden Verlust an Knochenmasse bedingt, der auch durch eine zunehmende Verformung des Knochens begleitet sein kann. Diese Verformung empfindet der Patient als stechenden manchmal auch dumpfen, bohrenden Schmerz.

Bei der Vertebroplastie (bzw. Osteoplastie) wird der Verlust an Knochenmasse durch das Einspritzen von zunächst zähflüssigem Knochenzement wieder ausgeglichen. Die perkutane Vertebroplastie ist ein effektives neues interventionelles Verfahren zur Behandlung von Knochenschmerzen. Durch diese neue Behandlungsmethode, die bereits mit Erfolg in Frankreich und den USA angewendet wird, kann eine Stabilisierung des betroffenen Knochens und eine deutliche Schmerzreduktion erreicht werden. Die Verwendung von Knochenzement hat sich in der Endoprothetik bereits etabliert. Auch langfristige Untersuchungen haben gezeigt, dass bei stabiler Implantatlage spongiöser Knochen auch in der Zementeinbettung durchaus vital bleiben kann. Selbst die Auffüllung von Wirbelkörpern mit Knochenzement ist im Rahmen perkutaner interventioneller Methoden bereits mehrfach bewiesen, wobei die Schmerzreduktion dabei eine wesentliche Rolle spielt. Unter röntgenologischer und/oder computer-tomographischer Kontrolle wird der entsprechende Knochen mittels Applikationsset punktiert. Dies geschieht unter Lokalanästhesie meist in Kombination mit einer Neuroleptanalgesie. Wenn die Nadel im Bereich der pathologischen Fraktur bzw. des Knochentumors platziert ist, wird über diesen Weg niedrigvisköser Knochenzement unter permanenter Durchleuchtung in den Knochen eingebracht. Der Zement härtet nach kurzer Zeit aus und verleiht dem Knochen neue Stabilität. Während der Intervention erfolgt eine Überwachung des Patienten hinsichtlich Blutdruck, Sauerstoffsättigung und Schmerzsymptomatik.

Grundsätzlich muss nun davon ausgegangen werden, dass verschiedene Möglichkeiten der Zufuhr dieses Knochenzementes gemäß der diesseitigen Behandlungsmethode gegeben sind. Aus dem Stand der Technik sind diverse Applikationsvorrichtungen zur Einbringung des Knochenzementes für die beschriebene Behandlungsmethode bekannt. Dabei werden Vorrichtungen zum Applizieren von Knochenzement mit einem Gehäuse, das einen Zylinder zur Aufnahme des Knochenzementes umfasst, und mit einem in dem Zylinder längs verschiebbaren angeordneten Kolben, der durch den Knochenzement durch eine in den Zylinder ausgebildete Austrittsöffnung herauspressbar ist, wobei der Kolben zum Applizieren des Knochenzementes unter hohem Druck durch eine Schraubbewegung in den Zylinder längs verschiebbar ist, beschrieben. Es wird die entsprechende Vorrichtung an der vorgesetzten Kanüle in den zu behandelnden Knochen über eine an sich bekannte Luerlock-Verbindung mit der Applikationsvorrichtung verbunden.

Bei der Applikation sind mehrere Anforderungen zu beachten. Einerseits muss die Befüllung der Applikationsvorrichtung sowie die Applikation in die betroffenen Knochenstrukturen sehr schnell innerhalb weniger Minuten erfolgen, da die üblicherweise verwendeten Knochenzemente 6 bis 7 Minuten nach dem Anrühren auszuhärten beginnen. Andererseits muss der Knochenzement mit hoher Viskosität und sehr hohem Druck appliziert werden, da ansonsten eine ausreichende Durchdringung der Knochenstrukturen nicht gewährleistet ist. Letztlich muss die Applikation des Knochenzementes so gut steuerbar sein, da insbesondere bei der Applikation im Bereich der Wirbelsäule ein Fehlleiten des Knochenzementes zu irreversiblen Schädigungen, beispielsweise der Verschluss von Gefäßen und daraus folgend eine Embolie, führen kann.

Weiterhin ist bekannt die Patentschrift DE 100 64 202 "Vorrichtung zum Applizieren von Knochenzement und Kanüle für eine solche Vorrichtung", wobei eine Applikationsvorrichtung beschrieben ist, die ein Auffüllen des Zylinders durch eine Aufziehbewegung des Kolbens, das heißt in eine direkte Verschiebung des Kolbens in Längsrichtung, in sehr kurzer Zeit möglich macht. In umgekehrter Weise kann anschließend der im Zylinder vorhandene flüssige Knochenzement durch direktes Verschieben des Kolbens in kurzer Zeit solange appliziert werden, bis der entstehende Gegendruck so groß wird, dass er durch die direkte Vorschubbewegung nicht mehr überwunden werden kann. In diesem Moment wird die Applikationsvorrichtung auf einen Modus - Verschiebung des Kolbens durch Schraubbewegung - umgestellt, da durch die Schraubbewegung ein wesentlich höherer Druck auf den Kolben und damit auf den zu applizierenden Knochenzement ausgeübt werden kann als mit einer direkten Vorschubbewegung.

Gerade diese Anwendbarkeit einer Schraubverbindung, welche in verschiedenen Applikationsvorrichtungen für Knochenzement bekannt ist, liegt der wesentliche Nachteil des gegebenen Standes der Technik. Gerade durch die Anwendung dieser Schraubbewegung ist ein direkter Zusammenhang der Kraftübertragung beim Applizieren des Knochenzementes durch den bedienenden Arzt hin zum Austritt des Knochenzementes nicht gegeben. Der anwendende Arzt hat durch diese Kraftübertragung von Gewinden oder anderen kraftbeeinflussenden Getrieben nicht die Möglichkeit, den direkten Zusammenhang zwischen Knochenzement und Druckbelastung zu kontrollieren.

Des Weiteren besteht ein Nachteil bei der Applikationsvorrichtung gemäß der diesseitig genannten Lösung DE 100 64 202, dass eine zusätzliche Verlängerung auf die Applikationsvorrichtung angeschraubt werden muss, um zu verhindern, dass der behandelnde Arzt einer Bestrahlung unter röntgenologischer und/oder computer-tomographischer Kontrolle ausgesetzt ist, da er beim Applizieren des Knochenzementes mit einer entsprechenden Vorrichtung in den Strahlenbereich gelangt.

Eine Injektionspumpe gemäß dem Oberbegriff des Anspruchs 1 ist in der Druckschrift US-B-6395007 gezeigt.

Es ist die Aufgabe der vorliegenden Erfindung, eine Injektionspumpe zum Applizieren von hochviskosen Medien, welche mit hohem Druck appliziert werden müssen, insbesondere zur Anwendung bei perkutaner Vertebroplastie, anzugeben, mit der die Applikation in kurzer Zeit durchgeführt werden kann, wobei gleichzeitig der erforderliche hohe Druck aufgebaut werden kann und eine Anwendung unter röntgenologischer und/oder computer-tomographischer Kontrolle ohne eine Strahlenbelastung des behandelnden Arztes gewährleistet ist.

Die Aufgabe wird ausgehend von einer Injektionspumpe der eingangs genannten Art erfindungsgemäß dadurch gelöst, dass die Injektionspumpe zum Applizieren von hochviskosen Medien, welche mit hohem Druck appliziert werden müssen, insbesondere zur Anwendung bei perkutaner Vertebroplastie, gemäß des Anspruches 1 und dessen Unteransprüche ausgeführt wird. Dabei wurde eine Injektionspumpe geschaffen, welche nach einem an sich bekannten Pumpenprinzip arbeitet. Es wurde ein langer Pumpenkörper mit einem geringen Flächenvolumen ausgeführt, um somit eine geringe Kraftanstrengung beim Ausdrücken des hochviskosen Mediums aus der distalen Öffnung der Injektionspumpe zu erwirken.

Grundsätzlich kann bei der Beschreibung davon ausgegangen werden, dass im Rahmen dieser Anmeldung der Begriff "proximal" mit der Bedeutung "zum Körper des Arztes hin gelegen" und dementsprechend der Begriff "distal" mit der Bedeutung "vom Körper des Arztes entfernt gelegen" verwendet werden.

Bei der Injektionspumpe ist ein Kolbenstangengriff am proximalen Ende der Pumpe vorhanden, welcher eine starre Kolbenstange, die durch den Griff der Injektionspumpe bis in den Pumpenkörper reicht, aufweist. An dieser starren Kolbenstange ist eine flexible Kolbenstange angeordnet, welche sich somit dem gegebenen verformten oder flexiblen Pumpenkörper anpasst. Der Pumpenkörper ist so ausgeführt, dass er in einer starren Verformung vorgegeben ist oder durch die Verwendung von Kunststoffmaterialien flexibel und verformbar je nach Anwendungszweck einsetzbar ist. Die Länge der starren Kolbenstange mit der angekoppelten flexiblen Kolbenstange ist so ausgeführt, dass am Ende der flexiblen Kolbenstange ein Kolbenkopf am distalen Ende innerhalb des Pumpenkörpers abschließt. Des Weiteren ist am distalen Ende des Pumpenkörpers eine Schlauchbefestigungshülse und ein rotierbarer männlicher Luerlock vorhanden, welcher zum Anschluss an eine vom Arzt gesetzte Kanüle oder Nadel dienlich ist. Beim Aufsaugen des hochviskosen Mediums, zum Beispiel Knochenzement, mit der Injektionspumpe wird in diesen rotierbaren männlichen Luerlock eine Tülle eingeschraubt, um somit nach Auffüllung der Injektionspumpe mit einem hochviskosen Medium diese zu entfernen und einen sauberen Anschluss an eine entsprechende Kanüle zu gewährleisten. Die Länge des Pumpenkörpers ist so ausgeführt, dass der bedienende Arzt über den Kolbenstangengriff bei befüllter Injektionspumpe ohne Probleme das hochviskose Medium über die angeschlossene Kanüle mit geringer Kraftaufwendung einführen kann und somit das genaue Gefühl bei der Injektion des hochviskosen Mediums in den jeweiligen beschädigten Knochen durch die direkte Schubbewegung über den Kolbenstangengriff der starren Kolbenstange und der angeschlossenen flexiblen Kolbenstange bis hin zum Kolbenkopf ausführen kann. Darin ist ein wesentlicher Vorteil der beschriebenen Injektionspumpe gegeben, da ein direkter Zusammenhang zwischen Kraftanstrengung und Austritt des hochviskosen Mediums am distalen Ende der Injektionspumpe stattfindet. Der zu behandelnde Arzt hat in jedem Fall die Möglichkeit, die Führung durch seine Kraftanstrengung und den damit hervorgerufenen Austritt des hochviskosen Mediums am distalen Ende der Injektionspumpe selbst festzulegen und somit ein besseres Gefühl, diesen hochviskosen Medienaustritt zu steuern. Des Weiteren besteht die Möglichkeit, durch leichtes Zurückziehen des Kolbenstangengriffes bei der Injektion des hochviskosen Mediums in beschädigte Wirbelteile einen überschüssigen Austritt des bestehenden hochviskosen Mediums zu verhindern und somit eine Druckentlastung in dem Pumpensystem zu verursachen, wobei eine korrekte Platzierung des hochviskosen Mediums in den beschädigten Knochen ausführbar ist. Zu dem am distalen Ende befindlichen Kolbenkopf ist weiterhin eine technische Lösung gegeben, welche eine Ventilwirkung dahingehend aufweist, dass beim Einpressen des hochviskosen Mediums ein Austritt der Luft, welche im Pumpenkörper vorhanden ist, stattfindet. Der Kolbenkopf ist so ausgeführt, dass er mittig eine Bohrung aufweist, welche im hinteren Teil mit einem Filter, zum Beispiel Zellulose oder Schaumstoff, ausgefüllt ist, die dahingehend luftdurchlässig ist. Wird nun das beinhaltete hochviskose Medium in dem Pumpenkörper über die Kraftanwendung des bedienenden Arztes ausgedrückt, entsteht ein Überdruck, welcher über den Filter und einer senkrechten Bohrung im Kolbenkopf austreten kann. Über dieser senkrechten Bohrung ist weiterhin ein Ventilschlauch angeordnet, welcher somit eine Ventilwirkung für den Austritt der Luft beim Einpressen des hochviskosen Mediums darstellt.

Die Erfindung wird nachstehend an einem Ausführungsbeispiel näher erläutert, wobei vier Zeichnungen gegeben sind.
- Figur 1: zeigt die Injektionspumpe;
- Figur 2: zeigt die Ausführung des Pumpenkörpers;
- Figur 3: zeigt das distale Ende des Pumpenkörpers;
- Figur 4: zeigt das Ansaugen und Ausdrücken des Knochenzementes im Pumpenkörper.

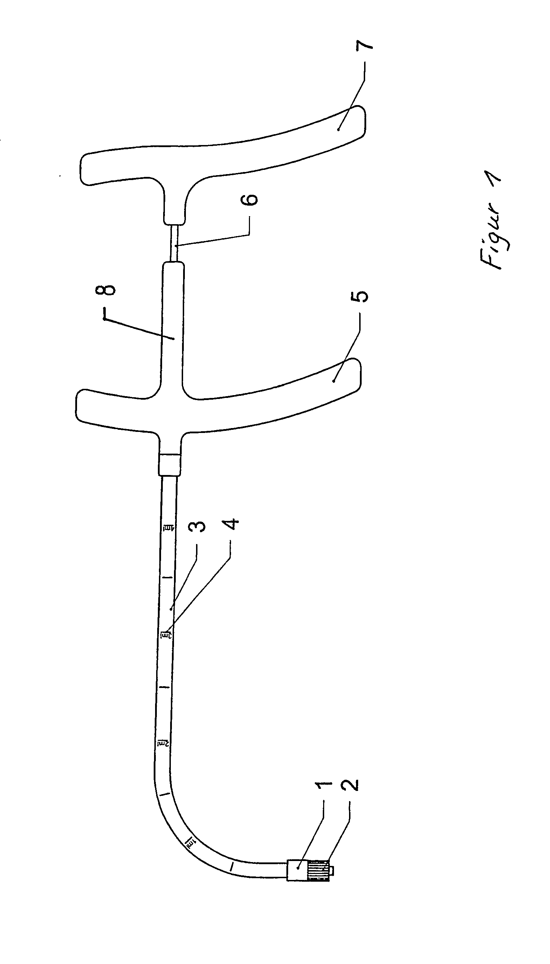

Figur 1 zeigt eine Injektionspumpe 8 in einer normalen Ansicht, wobei sich die Injektionspumpe 8 zusammensetzt aus einem Kolbenstangengriff 7 der am distalen Ende befestigten starren Kolbenstange 6, welche durch einen Griff 5 der Injektionspumpe 8 hinein in den Pumpenkörper 3 verschiebbar angeordnet ist. Dabei ist der Pumpenkörper 3 entweder in einer Art und Weise vorgegeben, dass dieser schon vorgeformt starr ausgefertigt ist, bzw. kann der Pumpenkörper 3 auch ein entsprechender Kunststoffschlauch flexibel verformbar ausgestaltet sein. Dabei sind auf dem Pumpenkörper 3 ml-Markierungen 4 angeordnet, um somit den Knochenzementinhalt zu deklarieren. Am distalen Ende des Pumpenkörpers 3 ist eine Schlauchbefestigungshülse 1 im Zusammenhang mit einem rotierbaren männlichen Luerlock 2 angeordnet. Dieser rotierbare männliche Luerlock 2 dient zum Anschluss an eine gesetzte Kanüle bzw. Nadel, welche der Arzt vorher mit entsprechenden Hilfsmitteln in den zu behandelnden Knochen platziert. Die Ausführungsvariante gemäß der Figur 1 zeigt eine Darstellung der Injektionspumpe 8, wobei entsprechende Griffstücke 5 bzw. 7 so ausgeformt sind, dass sie eine leichte Handhabung der jeweiligen Injektionspumpe 8 gewährleisten. Die zeichnerische Darstellung der Figur 1 zeigt die Injektionspumpe 8 in einem Ausgangszustand, worin noch kein Knochenzement 17 in den Pumpenkörper 3 aufgesaugt wurde. Es lässt sich also feststellen, dass insbesondere die starre Kolbenstange 6 im geschlossenen Zustand einen gewissen Teil aus dem Griff 5 herausragt. Grundsätzlich kann man davon ausgehen, dass gerade diese starre Kolbenstange 6 zum distalen Ende hin in jedem Fall zum Beispiel mindestens einen Zentimeter in den Pumpenkörper 3 hineinragt, um somit eine entsprechende Stabilität auszuführen. Die starre Kolbenstange 6 ist vorzugsweise aus Metall gefertigt. Die Verbindung zwischen dem Pumpenkörper 3 und dem Griff 5 kann fest, rotierbar und auswechselbar ausgeführt werden. Durch die Herstellung des Pumpenkörpers 3 aus Kunststoff ist dieser flexibel formbar und je nach Anwendung in gefülltem Zustand mit Knochenzement so verbiegen, dass er problemlos auf eine platzierte Kanüle über den rotierbaren männlichen Luerlock 2 luftdicht anschließbar ist. Bei der Ausführung des Pumpenkörpers 3 sind auch Varianten möglich, dass dieser in einem vorgeformten Zustand ausgeführt ist, wobei an der starren Kolbenstange 6 am distalen Ende eine flexible Kolbenstange 9 angeordnet ist, die sich in jedem Fall an die Verformung des Pumpenkörpers 3 anpasst. Der Griff 5 ist in seiner vertikalen Länge ca. 10 cm ausgeführt. Des Weiteren ist der Pumpenkörper 3 an den Griff 5 in einer Länge von ca. 22 cm ausgeführt. Diese Größenordnungen können sich je nach Anwendung relativ dahingehend verändern, dass der Pumpenkörper 3 kürzer bzw. länger ausgefertigt ist. Weiterhin sind verschiedene Griffformen des Kolbenstangengriffes 7 möglich. Wie in der Erfindung schon beschrieben ragt die starre Kolbenstange 6 von Seiten des Kolbenstangengriffes 7 über den Griff 5 hin bis in den Pumpenkörper 3 hinein, wobei zwei Drittel der Gesamtlänge der Injektionspumpe 8 als vorteilhaft erscheinen. Die restliche Größenordnung wird anschließend mit der flexiblen Kolbenstange 9 versehen, welche sich dann an vorgeformte Biegungen bzw. einen Kunststoffpumpenkörper 3 anpasst.

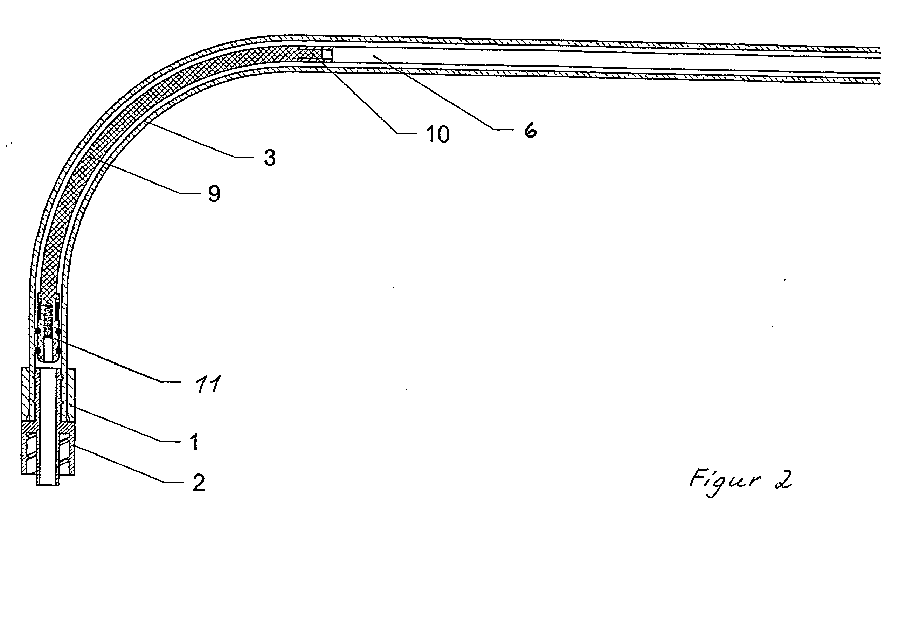

Figur 2 zeigt insbesondere den Pumpenkörper 3 in seinem inneren Aufbau. Dabei ist ersichtlich, dass die starre Kolbenstange 6 vor einer etwaigen Biegung endet und anschließend an dieser starren Kolbenstange 6 eine flexible Kolbenstange 9 über eine entsprechende Verbindung der beiden Kolbenstangen 10 angeordnet ist. Die flexible Kolbenstange 9 ist vorteilhafterweise aus Kunststoff gefertigt, kann aber auch aus Federstahl und anderen biegsamen, festen Materialien hergestellt sein, womit eine Formgebung an den vorgeformten bzw. flexiblen Pumpenkörper möglich ist. Am distalen Ende der flexiblen Kolbenstange 9 ist ein Kolbenkopf 11 in zusammengeschobenem Zustand angeordnet, welcher unmittelbar vor der Schlauchbefestigungshülse 1 mit nachfolgendem rotierbaren männlichen Luerlock 2 endet. Dieser Kolbenkopf 11 ist über Dichtringe 13 so ausgefertigt, dass eine Saugwirkung zum Hereinziehen von Knochenzement entsteht. Vorteilhafterweise ist der Pumpenkörper 3 in einer besonderen Ausführungsform ca. 20 bis 25 cm lang und aus Kunststoff gefertigt, womit ein flexibel geformter Pumpenkörper 3 über den distal angeordneten rotierbaren männlichem Luerlock 2 durch den Arzt anschließbar auf eine gesetzte Kanüle ist.

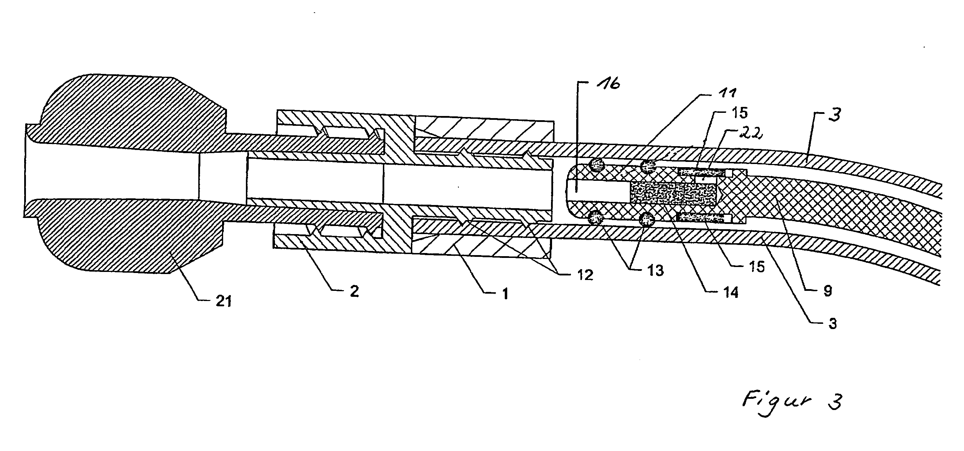

Die Figur 3 zeigt das distale Ende der Injektionspumpe 8, insbesondere den klaren Aufbau des Kolbenkopfes 11 am distalen Ende der flexiblen Kolbenstange 9, sowie die Anordnung der Schlauchbefestigungshülse 1 mit anliegendem rotierbaren männlichen Luerlock 2 und einer Tülle 21, welche in das rotierbare männliche Luerlock 2 eingeschraubt wird und als Tülle zum Ansaugen von Knochenzement 17 aus einem Behältnis dient. Diese Tülle 21 wird nach Vollsaugen der Injektionspumpe 8 mit Knochenzement 17 in der vorgegebenen Menge aus dem rotierbaren männlichen Luerlock 2 herausgeschraubt, womit gewährleistet ist, dass das rotierbare männliche Luerlock 2 auf eine Luerlock-Verbindung sauber aufgesetzt werden kann. Wichtig ist dabei, dass die Verbindungen zwischen dem Pumpenkörper 3, der Schlauchbefestigungshülse 1 und dem darin befindlichen rotierbaren männlichen Luerlock 2 luftdicht angeordnet sind, damit beim Ansaugen bzw. beim Hereinpressen des Knochenzementes 17 keine Luft angesaugt wird.

In der besonderen Ausführungsvariante der technischen Lösung ist das männliche Luerlock 2 rotierbar und so angeordnet, dass die Dichtheit gewährleistet ist, dass in dem männlichen Luerlock 2 die Schlauchbefestigung bzw. der Pumpenkörper 3 befestigt ist, in dem der Luerlock 2 mit Zacken 12 versehen ist, worin der Pumpenkörper 3 zur Befestigung in der Schlauchbefestigungshülse 1 radial eingepresst wird.

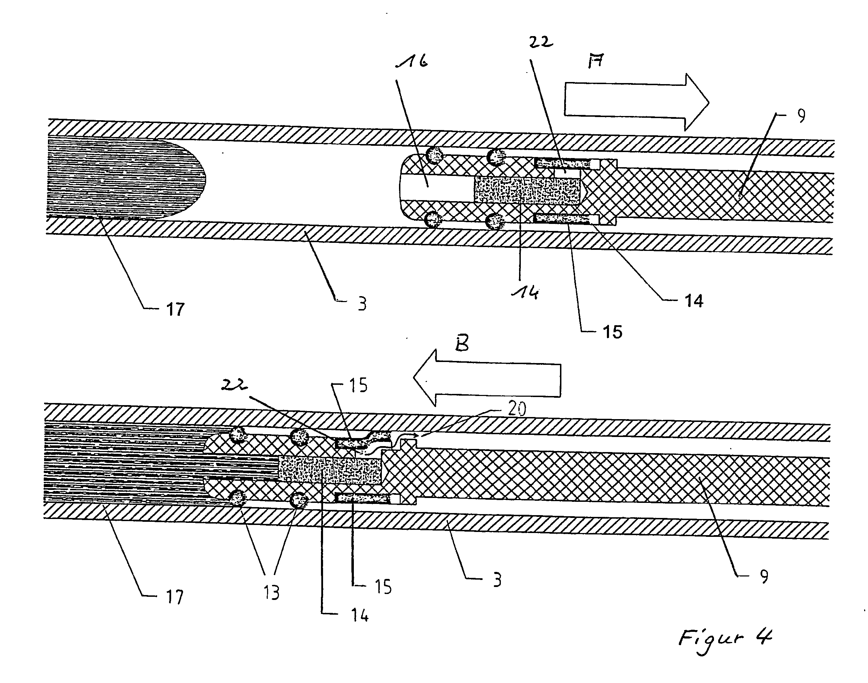

Sollte jedoch Luft beim Hereinziehen des Knochenzementes 17 in den Pumpenkörper 3 eindringen, wird durch eine extra vorgegebene Entlüftung am Kolbenkopf 11 diese entsorgt. Der Kolbenkopf 11 ist am distalen Ende der flexiblen Kolbenstange 9 angeordnet. Zwischen dem Kolbenkopf 11 und der Innenwand des Pumpenkörpers 3 sind jeweils Dichtringe 13 mit einem definierten Abstand doppelt angeordnet, um somit die Saugwirkung beim Ansaugen von Knochenzement 17 zu gewährleisten. Der Abstand ist so gewählt, dass bei einer Biegung des Pumpenkörpers 3 trotzdem die Luftdichtheit gewährleistet ist. Mittig innerhalb des Kolbenkopfes 11 ist eine Bohrung zur Entlüftung 16 gegeben. Diese Bohrung wird so ausgeführt, dass sie bis zwei Drittel der Länge vom distalen Ende an in den Kolbenkopf 11 hineinragt. Zur Hälfte wird diese Bohrung 16 mit Zellulose 14 ausgekleidet. Diese Zellulose 14 ist so ausgeführt, dass sie bei entsprechenden Druckverhältnissen durch leichten Überdruck von ca. 0,01 bar luftdurchlässig ist. Es sind aber auch andere Materialien, wie Schaumstoff oder luftdurchlässige, als Filter wirkende Materialien, denkbar. Am Ende der mittleren Bohrung 16 ist eine senkrechte Bohrung 22 in Verbindung mit der mittleren Bohrung 16 gegeben. Über diese senkrechte Bohrung 22 ist ein Ventilschlauch 15 radial angeordnet, welcher insbesondere bei bestimmten Druckverhältnissen über der Bohrung 22 flexibel ausgeführt ist. Grundsätzlich dient dieser Ventilschlauch 15 insbesondere zur Entlüftung des angesaugten Knochenzementes 17, was nachfolgend noch zur Figur 4 beschrieben wird.

Als weitere Ausführungsmöglichkeit ist gegeben, dass die beiden Dichtringe 13 und der Ventilschlauch 15 in einer gesonderten Bauart so ausgeführt sind, dass eine einzige Dichtung so angeordnet ist, dass ebenfalls auch eine Ventilschlauchwirkung erreicht wird. Somit entsteht eine Dichtungsmanschette, welche gleichzeitig eine Ventilwirkung zur Entlüftung darstellt.

Figur 4 zeigt eine Bewegungsrichtung "Ansaugen A" bzw. eine Bewegungsrichtung "Auspressen B" des Knochenzementes 17. Dabei ist bei der Bewegungsrichtung "Ansaugen A" ersichtlich, dass durch das Herausziehen der flexiblen Kolbenstange 9 aus den Pumpenkörper 3 mit anschließender starren Kolbenstange 6 über den Kolbenstangengriff 7 der Knochenzement 17 aus einem Behälter angesaugt wird. Wie aus der Zeichnung ersichtlich, entsteht je nach Handhabung der Injektionspumpe 8 ein bestimmtes Luftpolster zwischen dem Knochenzement 17 und dem distalen Ende der flexiblen Kolbenstange 9 bis hin zum Kolbenkopf 11. Diese entstehenden Luftbläschen und Luftpolster müssen nun nachfolgend aus dem Pumpenkörper 3 entfernt werden, um somit bei der Injektion des Knochenzementes 17 in den jeweiligen Knochen des zu behandelnden Knochensegmentes des Patienten über eine entsprechende gesetzte Kanüle keinen Lufteintritt zu verursachen. Dabei ist die Bewegungsrichtung "Auspressen B" gegeben, wobei die flexible Kolbenstange 9 mit dem Kolbenkopf 11 in distaler Richtung hin zum Knochenzement 17 bewegt wird. Durch den entstehenden Überdruck und der Dichtverbindung über die Dichtringe 13 bis in die mittlere Bohrung 16 zur Zellulose 14 kann nun über die Zellulose 14, der senkrechten Bohrung 22 und des sich öffnenden Ventilschlauches 15 ein Luftaustritt 20 gewährleistet werden. Somit kann man durch das Schieben der Kolbenstangen 9 und 6 in Richtung distales Ende erwirken, dass der beinhaltete Knochenzement 17, welcher vorher angesaugt wurde, entlüftet wird. Gerade aus der Zeichnung 4 ist ersichtlich, dass die störende Luft beim Aufziehen der Injektionspumpe 8 mit eingezogen wird, da das Material meist sehr zähflüssig in seiner Konsistenz ist, empfiehlt es sich auf Grundlage der Ventilwirkung, welche gerade an dem Kolbenkopf 11 beschrieben wurde, eine so genannte Entlüftung auszuführen.

Nachfolgend soll die Handhabung insbesondere für das diesseitig beschriebene Ausführungsbeispiel der Injektionspumpe 8 und die bestehenden Vorteile beschrieben werden. Bei der Vertebroplastie handelt es sich um ein neues Verfahren zur perkutanen Argumentation von Wirbelkörpern mit Knochenzement. Hierbei wird der geschwächte frakturierte Wirbelkörper stabilisiert und die Schmerzensymptomatik der Patienten entscheidend verbessert. In der Bauchlage wird unter Durchleuchtung mittels CT- bzw. MRT-Verfahren über den Pedikel der betroffene Wirbelkörper mit einer Knochenpunktionsnadel punktiert. Zusätzlich wird ein frisch angerührter, steriler und flüssiger Knochenzement (PMMA, Polymethyl-Methacrylate) injiziert. Dieser Zement ist im Wesentlichen der gleiche, der seit Jahrzehnten zum Einzementieren von Gelenkprothesen Verwendung findet. Nachfolgend wird über die Injektionspumpe 8 mit der Tülle 21 am distalen Ende auf die Schlauchbefestigungshülse 1 in Kombination mit dem rotierbaren männlichen Luerlock 2 der Knochenzement 17 aufgesaugt. Dabei ist die Tülle 21 bereits in das Luerlock 2 eingeschraubt. Ist die entsprechende Menge des Knochenzementes 17 in der Injektionspumpe 8 eingefügt, wird die Tülle 21 am distalen Ende der Injektionspumpe 8 von dem Luerlock 2 abgeschraubt. Durch leichtes Drücken der Pumpe in Richtung des distalen Endes kann über die Entlüftung an dem Kolbenkopf 11 die entsprechende mit eingezogene Luft herausgefiltert werden. Nachfolgend wird die Injektionspumpe 8 auf die Knochenpunktionsnadel über die Luerlock-Verbindung aufgesetzt. Die nachfolgende Injektion des Knochenzementes 17 über die Injektionspumpe 8 erfolgt ebenfalls im laufenden bildgebenden Verfahren, so dass die Zementeingabe gut kontrollierbar ist. Die wesentlichen Vorteile der Handhabung der Injektionspumpe 8 sind darin gegeben, dass hierbei eine flexible Anordnung über den flexiblen Pumpenkörper 3 oder vorgeformten Pumpenkörper 3 möglich ist, da insbesondere in bestimmten bildgebenden Verfahren ein sehr geringer Platz vorgegeben ist, um eine entsprechende Injektionspumpe 8 auf eine gesetzte Nadel aufzubringen. Insbesondere ist dabei zu erwähnen, dass in dem CT-Verfahren für eine Möglichkeit einer Aufsetzung der Injektionspumpe 8 nur ca. 10 bis 30 mm vorhanden sind. Des Weiteren ist auf Grund der Größe der Injektionspumpe 8 verhinderbar, dass der zu behandelnde Arzt in den Strahlenbereich des bildgebenden Verfahrens gelangt. Beim Applizieren des Knochenzementes 17 in der beinhalteten Injektionspumpe 8 ist auf Grund der positiv gegebenen Kraftverhältnisse, Länge der Kanüle und des Durchmessers der Kanüle eine gute Handhabung bei der Einpressung des Knochenzementes 17 über die Nadel ausführbar. Dabei hat der benutzende Arzt die Möglichkeit, durch den direkten Kontakt mit der Kraftwirkung auf den Kolbenstangengriff 7 beim Hereinpressen die Menge des eingebrachten Knochenzementes 17 über die Pumpenwirkung zu steuern. Außerdem ist es möglich, bei Einbringen des Knochenzementes 17 durch leichtes Zurückziehen über den Kolbenstangengriff 7 eine Druckentlastung zu erwirken. Wesentlich dabei ist auch, dass auf Grundlage der Injektionspumpe 8 und der flexiblen Ausführung eines Pumpenkörpers 3 eine leichte Anbringung auf eine entsprechende Nadel über die Luerlock-Verbindungen der beiden Instrumente möglich ist. Durch die vorgegebene Länge der Injektionspumpe 8 kann ohne Probleme in einem bildgebenden Verfahren gearbeitet werden.

### Bezugszeichen

- 1: Schlauchbefestigungshülse
- 2: rotierbarer männlicher Luerlock
- 3: Pumpenkörper
- 4: ml-Markierung auf Pumpenkörper
- 5: Griff für Injektionspumpe
- 6: starre Kolbenstange
- 7: Kolbenstangengriff der Injektionspumpe
- 8: Injektionspumpe
- 9: flexible Kolbenstange
- 10: Verbindung starre Kolbenstange mit flexibler Kolbenstange
- 11: Kolbenkopf
- 12: Zacken
- 13: Dichtringe
- 14: Zellulose
- 15: Ventilschlauch
- 16: Bohrung für Entlüftung
- 17: Knochenzement
- 20: Luftaustritt
- 21: Tülle
- 22: senkrechte Bohrung

## Patentansprüche

1. Injektionspumpe zum Applizieren von hochviskosen Medien, welche mit hohem Druck appliziert werden müssen, insbesondere zur Anwendung bei der perkutanen Vertebroplastie, wobei ein Kolbensystem mit Griffstücken zum Aufsaugen von Knochenzement in einen Kolben ausgeführt ist, **dadurch gekennzeichnet, dass** an einem Kolbenstangengriff (7) der Injektionspumpe (8) eine Kolbenstange (6) starr angeordnet ist und am distalen Ende der starren Kolbenstange (6) eine flexible Kolbenstange (9) bis hin zum distalen Ende eines Pumpenkörpers (3) mit am Ende gegebenem Kolbenkopf (11) vorhanden ist, wobei der Pumpenkörper (3) am proximalen Ende an einem Griff (5) der Injektionspumpe (8) befestigt ist.

2. Injektionspumpe nach Anspruch 1 **dadurch gekennzeichnet, dass** die Länge der starren Kolbenstange (6) so dimensioniert ist, dass bei herausgezogener Kolbenstange (6) über den Griff (5) mit Hilfe des Kolbenstangengriffes (7) die starre Kolbenstange (6) im Pumpenkörper (3) verbleibt.

3. Injektionspumpe nach Anspruch 1 **dadurch gekennzeichnet, dass** der Pumpenkörper (3) flexibel bzw. verformbar ausgeführt ist, wobei ein Kunststoff für den Pumpenkörper (3) vorteilhaft ist.

4. Injektionspumpe nach den Ansprüchen 1 und 3 **dadurch gekennzeichnet, dass** der Pumpenkörper (3) eine starre gebogene Ausrichtung aufweist.

5. Injektionspumpe nach Anspruch 3 **dadurch gekennzeichnet, dass** sich die flexible Kolbenstange (9) an der vorgegebenen starren Verformung des Pumpenkörpers (3) anpasst.

6. Injektionspumpe nach den Ansprüchen 3 und 4 **dadurch gekennzeichnet, dass** sich die flexible Kolbenstange (9) dem vorgeformten Pumpenkörper (3) in seiner Form anpasst.

7. Injektionspumpe nach den Ansprüchen 1, 5 und 6 **dadurch gekennzeichnet, dass** die flexible Kolbenstange (9) am Ende ein relativ weiches oder flexibles Material aufweist, vorzugsweise einen Kunststoff darstellt.

8. Injektionspumpe nach Anspruch 1 **dadurch gekennzeichnet, dass** am distalen Ende der flexiblen Kolbenstange (9) ein Kolbenkopf (11) innerhalb des Pumpenkörpers (3) angeordnet ist, wobei Dichtringe (13) zwischen dem Kolbenkopf (11) und dem Pumpenkörper (3) gegeben sind und somit eine Saugwirkung beim Herausziehen der Kolbenstangen (6 und 9) zur proximalen Richtung hin entsteht.

9. Injektionspumpe nach Anspruch 1 **dadurch gekennzeichnet, dass** am distalen Ende des Pumpenkörpers (3) eine Schlauchbefestigungshülse (1) mit einem anschließenden rotierbaren männlichen Luerlock (2) angeordnet ist.

10. Injektionspumpe nach Anspruch 9 **dadurch gekennzeichnet, dass** auf dem rotierbaren männlichen Luerlock (2) eine Tülle (21) für die Aufsaugung von hochviskosen Medien aus einem entsprechenden Gefäß eingedreht ist und diese nach Aufsaugen der hochviskosen Medien schraubbar entfernbar ist.

11. Injektionspumpe nach Anspruch 8 **dadurch gekennzeichnet, dass** der Kolbenkopf (11) an der flexiblen Kolbenstange (9) mittig eine Bohrung zur Entlüftung (16) aufweist, wobei in dem hinteren Teil der Bohrung (16) ein Filter, vorzugsweise aus Schaumstoff bzw. Zellulose (14), vorhanden ist, welcher luftdurchlässig wirkt.

12. Injektionspumpe nach den Ansprüchen 8 und 11 **dadurch gekennzeichnet, dass** eine senkrechte Bohrung (22) am proximalen Ende der mittleren Bohrung zur Entlüftung (16) im Kolbenkopf (11) vorhanden ist und diese senkrechte Bohrung (22) mit einem Ventilschlauch (15) radial überdeckt ist.

13. Injektionspumpe nach Anspruch 9 **dadurch gekennzeichnet, dass** an dem männlichen Luerlock (2) zur Befestigung des Pumpenkörpers (3) Zacken (12) gegeben sind, womit der Pumpenkörper (3) radial eingepresst ist.

14. Injektionspumpe nach Anspruch 1 **dadurch gekennzeichnet, dass** der Pumpenkörper (3) am Griff (5) fest, rotier- bzw. auswechselbar angeordnet ist.

## Claims

1. Injection pump for application of highly viscous media that have to be applied with pressure, in particular during percutaneous vertebroplasty, in which a piston system with grip ends to take up bone cement is provided in a piston, **characterised in that** a piston rod (6) is rigidly arranged at a piston rod grip (7) of the injection pump (8) and the distal end of the rigid piston rod (6) is provided with a flexible piston rod (9) to the distal end of a pump body (3) with an end piston head (11), where the pump body (3) is fastened at the proximal end at a grip (5) of the injection pump (8).

2. Injection pump according to claim 1, **characterised in that** the length of the rigid piston rod (6) being dimensioned in such manner that the rigid piston rod (6) remains in the pump body (3) when the piston rod (6) is pulled out through the grip (5) by means with the piston rod grip (7).

3. Injection pump according to claim 1, **characterised in that** the pump body (3) is flexible or ductile with preferable use of a plastic material for the pump body (3).

4. Injection pump according to claims 1 and 3, **characterised in that** the pump body (3) has a rigidly bent shape.

5. Injection pump according to claim 3, **characterised in that** the flexible piston rod (9) is matched to the chose rigid deformation of the pump body (3).

6. Injection pump according to claims 3 and 4, **characterised in that** the flexible piston rod (9) is matched to the shape of the pre-formed pump body (3).

7. Injection pump according to claims 1, 5 and 6, **characterised in that** the flexible piston rod (9) is fitted at its end with a relatively soft or flexible material, preferably a plastic material.

8. Injection pump according to claim 1, **characterised in that** a piston head (11) is arranged in the pump body (3) at the distal end of the flexible piston rod (9), with sealing rings (13) between piston head (11) and pump body (3) to create a suction effect when pulling out the piston rods (6 and 9) in proximal direction.

9. Injection pump according to claim 1, **characterised in that** a hose bracket sleeve (1) with an attached rotatable male LuerLock (2) at the distal end of the pump body (3).

10. Injection pump according to claim 9, **characterised in that** a nozzle (21) is screwed to the rotatable male LuerLock (2) to take up highly viscous media from a respective vessel which nozzle (21) can be unscrewed after absorption of such highly viscous media.

11. Injection pump according to claim 8, **characterised in that** the piston head (11) at the flexible piston rod (9) has a centred venting boring (16), with the rear section of the boring (16) being equipped with an air-permeable filter, preferably of foam material or cellulose (14).

12. Injection pump according to claims 8 and 11, **characterised in that** the proximal end of the centred venting boring (16) in the piston head (11) is provided with a vertical boring (22), which vertical boring (22) is radially covered with a valve hose (15).

13. Injection pump according to claim 9, **characterised in that** the male LuerLock (2) is fitted with a prong (12) to fasten the pump body (3) by radially pressure-forcing the pump body (3) into place.

14. Injection pump according to claim 1, **characterised in that** the pump body (3) is arranged at the grip (5) firmly, rotatable and replaceable.

## Revendications

1. Pompe à injecter destinée à l'application de fluides très visqueux devant être appliqués à une pression élevée, trouvant notamment son application dans le cas de la vertébroplastie percutanée, un système de piston muni d'éléments de préhension destiné à aspirer du ciment osseux étant réalisé dans un piston, **caractérisée en ce qu'**une tige de piston (6) est disposée fixement sur l'une des poignées de tige de piston (7) de la pompe à injecter (8) et une tige de piston souple (9) s'étend à l'extrémité distale de la tige de piston fixe (6) jusqu'à l'extrémité distale d'un corps de pompe (3) muni d'une tête de piston (11) disposée au bout, le corps de pompe (3) étant fixé à l'extrémité proximale à une poignée (5) de la pompe à injecter (8).

2. Pompe à injecter selon la revendication 1, **caractérisée en ce que** la longueur de la tige de piston fixe (6) est dimensionnée de sorte que la tige de piston fixe (6) reste à l'intérieur du corps de pompe (3) lorsque la tige de piston (6) sort de la poignée (5) à l'aide de la poignée de tige de piston (7).

3. Pompe à injecter selon la revendication 1, **caractérisée en ce que** le corps de pompe (3) est réalisé de façon à être souple ou déformable, une matière plastique étant avantageuse pour la composition du corps de pompe (3).

4. Pompe à injecter selon les revendications 1 et 3, **caractérisée en ce que** le corps de pompe (3) présente une forme courbe et souple.

5. Pompe à injecter selon la revendication 3, **caractérisée en ce que** la tige de piston souple (9) est adaptée à la déformation fixe prédéfinie du corps de pompe (3).

6. Pompe à injecter selon les revendications 3 et 4, **caractérisée en ce que** la forme de la tige de piston souple (9) est adaptée à celle du corps de pompe préformé (3).

7. Pompe à injecter selon les revendications 1, 5 et 6, **caractérisée en ce que** la tige de piston souple (9) présente à son extrémité un matériau souple ou relativement mou, de préférence une matière plastique.

8. Pompe à injecter selon la revendication 1, **caractérisé en ce qu'**une tête de piston (11) est disposée à l'intérieur du corps de pompe (3) à l'extrémité distale de la tige de piston souple (9), des bagues d'étanchéité (13) étant disposées entre la tête de piston (11) et le corps de pompe (3), provoquant ainsi une effet d'aspiration lorsque les tiges de piston (6 et 9) sont retirées dans le sens proximal.

9. Pompe à injecter selon la revendication 1, **caractérisée en ce qu'**un manchon de fixation pour tuyaux (1) muni d'un raccord Luer-Lock (2) mâle rotatif est disposé à l'extrémité distale du corps de pompe (3).

10. Pompe à injecter selon la revendication 9, **caractérisée en ce qu'**un embout (21) destiné à aspirer des fluides très visqueux d'un contenant prévu à cet effet est vissé sur le Luer-Lock mâle rotatif (2) et peut être enlevé par dévissage une fois les fluides très visqueux aspirés.

11. Pompe à injecter selon la revendication 8, **caractérisée en ce que** la tête de piston (11) de la tige de piston souple (9) présente au centre un perçage d'évacuation de l'air (16), un filtre, de préférence en mousse ou en cellulose (14), laissant passer l'air, étant disposé dans la partie arrière du perçage (16).

12. Pompe à injecter selon les revendications 8 et 11, **caractérisée en ce qu'**un perçage vertical (22) est disposé à l'intérieur de la tête de piston (11) à l'extrémité proximale du perçage médian d'évacuation de l'air (16) et ce perçage vertical (22) est recouvert radialement par un tuyau à valve (15).

13. Pompe à injecter selon la revendication 9, **caractérisée en ce que** des dents (12) permettant de presser radialement le corps de pompe (3) sont disposées sur le Luer-Lock mâle (2) afin de fixer le corps de pompe (3).

14. Pompe à injecter selon la revendication 1, **caractérisée en ce que** le corps de pompe (3) est disposé sur la poignée (5) de façon fixe, à pouvoir tourner ou être remplacé.
